(19) Europäisches Patentamt / European Patent Office / Office européen des brevets

(11) **EP 3 357 484 A1**

(12) **EUROPEAN PATENT APPLICATION**
published in accordance with Art. 153(4) EPC

(43) Date of publication:
08.08.2018 Bulletin 2018/32

(51) Int Cl.:
*A61K 8/35* (2006.01)       *A61K 8/37* (2006.01)
*A61K 8/39* (2006.01)       *A61Q 17/04* (2006.01)

(21) Application number: 16851845.4

(22) Date of filing: 30.09.2016

(86) International application number:
PCT/JP2016/079011

(87) International publication number:
WO 2017/057676 (06.04.2017 Gazette 2017/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 30.09.2015 JP 2015195087

(71) Applicant: Shiseido Company Ltd.
Chuo-ku
Tokyo 104-0061 (JP)

(72) Inventors:
• YAMAKI, Satoshi
Yokohama-shi
Kanagawa 224-8558 (JP)
• NAGAI, Kouichi
Yokohama-shi
Kanagawa 224-8558 (JP)
• SUGIYAMA, Yuki
Yokohama-shi
Kanagawa 224-8558 (JP)

(74) Representative: Ter Meer Steinmeister & Partner
Patentanwälte mbB
Nymphenburger Straße 4
80335 München (DE)

(54) **SUNSCREEN COSMETIC**

(57) The purpose of the present invention is to provide a sunscreen cosmetic capable of stably exerting an excellent ultraviolet shielding effect without undergoing photodegradation even in a system where 4-tert-butyl-4'-methoxydibenzoylmethane and ethylhexyl methoxycinnamate coexist. Provided is a sunscreen cosmetic comprising (a) 0.5-5.0 mass% of 4-tert-butyl-4'-methoxydibenzoylmethane, (b) 3.0-10 mass% of ethylhexyl methoxycinnamate, (c) 5.0 mass% or more of an amphipathic substance, and (d) a liquid oil component containing an ester oil having an IOB of 0.05-0.60, characterized in that the contents of components (a), (b) and (c) satisfy the requirement $0.5 \leq [(a)+(b)]/(c) \leq 3.5$.

**Description**

TECHNICAL FIELD

The present invention relates to a sunscreen cosmetic. More specifically, the present invention relates to a sunscreen cosmetic that limits the photodegradation of ultraviolet ray absorbing agents and that stably achieves high ultraviolet ray protection performance across a wide wavelength range spanning from the UVA to the UVB ranges, while also having excellent properties when used.

BACKGROUND ART

Protecting the skin from damage due to ultraviolet rays is an important problem in skin care and body care, and various UV-care cosmetics for minimizing the harmful effects of ultraviolet rays on the skin have been developed. Sunscreen cosmetics, which are a type of UV-care cosmetic, are cosmetics that are intended to protect the skin from damage due to ultraviolet rays by covering the skin with a coating that contains an ultraviolet ray absorbing agent or an ultraviolet ray scattering agent, thereby absorbing or scattering UVA and UVB rays, and limiting the amount of ultraviolet rays that reach the skin (Non-Patent Document 1).

Sunscreen cosmetics are formulated by blending appropriate combinations of ultraviolet ray absorbing agents that chemically absorb ultraviolet rays and ultraviolet ray scattering agents that physically reflect/scatter ultraviolet rays so as to achieve good ultraviolet ray protection effects. Ultraviolet ray absorbing agents are organic compounds having structures that absorb the energy of ultraviolet rays, and can be classified into UVA absorbing agents, UVB absorbing agents and wide-range absorbing agents having absorption bands spanning from the UVA to the UVB wavelength ranges, depending on the absorption wavelength range.

Dibenzoylmethane derivatives such as 4-tert-butyl-4'-methoxy dibenzoylmethane are commonly used in cosmetics and the like as UVB absorbing agents having a maximum absorption wavelength at approximately 360 nm, but it is known that they have poor photostability and that their ultraviolet ray protection effects are lowered by photoirradiation (Patent Document 1). Patent Document 1 describes that the photostability is significantly increased by blending an alkylaryl-1,3-propanedione silicone derivative together with 4-(1,1-dimethylethyl)-4'-methoxy dibenzoylmethane, which is a dibenzoylmethane derivative. However, the aforementioned photostabilization effects are confirmed for only specific dibenzoylmethane derivatives.

On the other hand, in order to obtain good ultraviolet ray protection effects across a wide wavelength range spanning from the UVA to the UVB ranges, it is necessary to use a combination of a UVA absorbing agent and a UVB absorbing agent, but there was a problem in that photodegradation occurs when using a combination of a dibenzoylmethane derivative, which is a UVB absorbing agent, and ethylhexyl methoxycinnamate, which is a UVA absorbing agent.

Patent Document 2 discloses that the photodegradation of 4-tert-butyl-4'-methoxy dibenzoylmethane and ethylhexyl methoxycinnamate was suppressed by blending in tris(polypropylene glycol-3 benzyl ether) citrate, but the effects are not confirmed in a sunscreen cosmetic containing both ultraviolet ray absorbing agents.

RELATED ART DOCUMENTS

PATENT DOCUMENTS

Patent Document 1: JP 2007-106701 A
Patent Document 2: JP 5540243 B
NON-PATENT DOCUMENTS

Non-Patent Document 1: Shin-keshohingaku, 2nd edition, edited by Takeo Mitsui, 2001, published by Nanzando, pp. 497-504.

SUMMARY OF THE INVENTION

PROBLEMS TO BE SOLVED BY THE INVENTION

Thus, the problem addressed by the present invention is to provide a sunscreen cosmetic that does not undergo photodegradation even in a system in which 4-tert-butyl-4'-methoxy dibenzoylmethane and ethylhexyl methoxycinnamate coexist, and that can stably achieve high ultraviolet ray protection effects.

MEANS FOR SOLVING THE PROBLEMS

As a result of performing diligent research towards solving the above-mentioned problem, the present inventors discovered that, by blending an amphiphilic substance and a specific oil into a sunscreen cosmetic containing 4-tert-butyl-4'-methoxy dibenzoylmethane and ethylhexyl methoxycinnamate, the photodegradation of the ultraviolet ray absorbing agents can be effectively prevented, and a sunscreen cosmetic that stably achieves high ultraviolet ray protection effects (high SPF) across a wide wavelength range spanning from the UVA to the UVB ranges can be obtained, thereby completing the present invention.

In other words, the present invention provides a sunscreen cosmetic comprising:

(a) 0.5 to 5.0 mass% of 4-tert-butyl-4'-methoxy dibenzoylmethane;
(b) 3 to 10 mass% of ethylhexyl methoxycinnamate;
(c) 5.0 mass% or more of an amphiphilic substance; and
(d) an oil containing an ester oil having an IOB of 0.05 to 0.60;

wherein the blended amounts of the ingredients (a), (b) and (c) fulfill the following expression:

$$[(a) + (b)]/(c) = 0.5 \text{ to } 3.5$$

EFFECTS OF THE INVENTION

In the sunscreen cosmetic of the present invention, the photodegradation of the ultraviolet ray protection performance can be limited even when containing the maximum possible amounts of 4-tert-buty;-4'-methoxy dibenzoylmethane and ethylhexyl methoxycinnamate, so high ultraviolet ray protection effects across a wide wavelength range spanning from the UVA to the UVB ranges can be achieved over a long period of time. Furthermore, the sunscreen cosmetic of the present invention also has excellent storage properties and properties when used.

MODES FOR CARRYING OUT THE INVENTION

The sunscreen cosmetic of the present invention comprises, as essential ingredients, (a) 4-tert-butyl-4'-methoxy dibenzoylmethane, (b) ethylhexyl methoxycinnamate, (c) an amphiphilic substance, and (d) an ester oil having an IOB of 0.05 to 0.60. The ingredients will be explained in detail below.

(a) 4-Tert-butyl-4' -methoxy dibenzoylmethane

4-Tert-butyl-4'-methoxy dibenzoylmethane is a benzophenone-based ultraviolet ray absorbing agent having a maximum absorption wavelength in the UVA range, which has conventionally been widely used in cosmetics and the like.

The 4-tert-butyl-4'-methoxy dibenzoylmethane used in the present invention may be a commercially available product, an example of which is the compound that is commercially available from DSM Nutrition Japan under the name "Parsol 1789".

The blended amount of ingredient (a), 4-tert-butyl-4'-methoxy dibenzoylmethane, contained in the sunscreen cosmetic of the present invention is 0.5 to 5.0 mass%, preferably 0.5 to 3.0 mass%, more preferably 1.0 to 2.5 mass%.

(b) Ethylhexyl methoxycinnamate

Ethylhexyl methoxycinnamate is a cinnamate-based ultraviolet ray absorbing agent having a maximum absorption wavelength in the UVB range. The ethylhexyl methoxycinnamate used in the present invention may be a commercially available product, an example of which is the compound that is commercially available from DSM Nutrition Japan under the name "Parsol MCX".

The blended amount of ingredient (b), ethylhexyl methoxycinnamate, contained in the sunscreen cosmetic of the present invention is 3.0 to 10.0 mass%, preferably 4.0 to 8.0 mass%, more preferably 5.0 to 7.5 mass%.

(c) Amphiphilic substance

The amphiphilic substance (ingredient (c)) used in the present invention is one or more substances chosen from among alkylene oxide derivatives and polyhydric alcohol derivatives.

As the alkylene oxide derivative in the present invention, it is preferable to use a polyoxyalkylene/polyoxyethylene copolymer dialkyl ether expressed by the following formula (I):

$$R_1O-[(AO)_m(EO)_n]-R_2 \qquad (I)$$

such that, in formula (I), AO represents an oxyalkylene group having 3 to 4 carbon atoms, specific examples being an oxypropylene group, an oxybutylene group, an oxyisobutylene group, an oxytrimethylene group and an oxytetramethine group, among which an oxypropylene group and an oxybutylene group are preferred. EO represents an oxyethylene group.

The symbol m represents the average number of moles of AO added, such that $1 \leq m \leq 70$, and preferably, $2 \leq m \leq 20$. The symbol n represents the average number of moles of EO added, such that $1 \leq n \leq 70$, and preferably, $2 \leq n \leq 20$.

The order of addition of AO and EO is not particularly limited. AO and EO may be added in the form of blocks so as to form a block copolymer, or may be randomly added so as to form a random copolymer. Block copolymers include not only copolymers with two blocks, but also those having three or more blocks. Preferably, a random copolymer is used.

The molecular weight of the polyoxyalkylene/polyoxyethylene copolymer dialkyl ether represented by formula (I) is preferably approximately 500 to 5000. The ratio [EO/(AO + EO)] of the amount of EO to the total amount of AO and EO in each molecule is preferably 20 to 80 mass%.

$R_1$ and $R_2$ each independently represent a hydrogen atom or a hydrocarbon group having 1 to 4 carbon atoms. Examples of hydrocarbon groups include methyl groups, ethyl groups, n-propyl groups, isopropyl groups, n-butyl groups, sec-butyl groups and tert-butyl groups. Methyl groups and ethyl groups are preferred.

The $R_1$ and $R_2$ in each molecule may be the same type of hydrocarbon group, a mixture of a hydrocarbon group and a hydrogen atom, or a mixture of multiple hydrocarbon groups having different numbers of carbon atoms. However, for each of $R_1$ and $R_2$, the ratio between the numbers of hydrocarbon groups and hydrogen atoms that are present should be such that the ratio (Y/X) of the number (Y) of hydrogen atoms to the number (X) of hydrocarbon groups is preferably 0.15 or lower, and more preferably 0.06 or lower.

Specific examples of polyoxyalkylene/polyoxyethylene copolymer dialkyl ethers that can be favorably used in the present invention include, but are not limited to, the following polyoxypropylene/polyoxyethylene copolymer dimethyl ethers:

POP(2) POE(9) dimethyl ether (random copolymer)
POP(4) POE(17) dimethyl ether (random copolymer)
POP(7) POE(14) dimethyl ether (random copolymer)
POP(9) POE(11) dimethyl ether (random copolymer)
POP(28) POE(55) dimethyl ether (random copolymer)
POP(41) POE(36) dimethyl ether (random copolymer)
POP(6) POE(3) dimethyl ether (random copolymer)
POP(3) POE(6) dimethyl ether (random copolymer)
POP(4) POE(8) dimethyl ether (random copolymer)
POP(11) POE(6) dimethyl ether (random copolymer)
POP(27) POE(14) dimethyl ether (random copolymer)

Examples of polyhydric alcohol derivatives that can be used as the amphiphilic substance (ingredient (c)) in the present invention include glycerin, diethylene glycol, dipropylene glycol, dibutylene glycol, polyethylene glycol (also denoted "PEG"), polypropylene glycol (also denoted "PPG") and polybutylene glycol (also denoted "PBG"), and they may be chosen from among those that can be used in external agents for use on the skin, such as cosmetics, provided that the molecular weight should preferably be 500 or higher, and the molecular weight should more preferably be 1000 or higher. Although the upper limit of the molecular weight is not particularly limited, those having a molecular weight, for example, of 4000 or lower, 3000 or lower, or 2500 or lower may be favorably used.

The amphiphilic substance (ingredient (c)) in the sunscreen cosmetic of the present invention is of one or more types chosen from among alkylene oxide derivatives and polyhydric alcohol derivatives, but it should preferably contain at least one alkylene oxide derivative, particularly a polyoxyalkylene/polyoxyethylene copolymer dialkyl ether, among which a polyoxypropylene/polyoxyethylene copolymer dimethyl ether is particularly preferred.

The blended amount of the amphiphilic substance (ingredient (c)) should be 5.0 mass% or more, preferably 5.0 to 10 mass%. If the blended amount is less than 5.0 mass%, then there may be cases in which sufficient photostability improvement effects cannot be obtained, and if more than 10 mass% is blended, then the emulsion stability and the properties when used may be affected.

The sunscreen cosmetic of the present invention is characterized in that the blended amounts of the (a) 4-tert-butyl-4'-methoxy dibenzoylmethane, the (b) ethylhexyl methoxycinnamate, and the (c) amphiphilic substance fulfill the following expression: [(a) + (b)]/(c) = 0.5 to 3.5. The blended amount ratio ([(a) + (b)]/(c)) is preferably 1.0 to 3.0, more preferably 1.0 to 2.5. If this ratio exceeds 3.5, then there are cases in which the photodegradation of the ultraviolet ray absorbing agents cannot be sufficiently limited, and if the ratio is less than 0.5, there is a tendency for the emulsion stability or the storage properties to be degraded.

(d) Liquid oil

The liquid oil (ingredient (d)) blended into the sunscreen cosmetic of the present invention is an oil that is liquid at ambient temperature and that contains an ester oil having an IOB of 0.05 to 0.60. The IOB value of the ester oil is preferably within the range of 0.07 to 0.55, more preferably 0.1 to 0.5.

The "IOB value (Inorganic/Organic Balance Value) of the ester oil" in the present invention is defined as the sum, over all of the ester oils contained in the cosmetic, of the IOB value of each ester oil multiplied by the proportion of the blended amount of that ester oil relative to the total blended amount of all ester oils. Accordingly, when a cosmetic contains only one ester oil, the IOB value of the ester oil in that cosmetic will be the same as the IOB value specific to that ester oil.

Examples of the ester oil that can be used in the present invention include cetyl 2-ethylhexanoate, triethylhexanoin, diisopropyl sebacate, isopropyl myristate, tripropylene glycol pivalate, pentaerythritol tetra-2-ethylhexanoate, diethylhexyl succinate, diheptylundecyl adipate, glyceryl diisostearate, di(phytosteryl/octyldodecyl) L-lauroyl glutamate, 2-decyl tetradecanol, diisostearyl malate, olive oil, meadowfoam oil, isocetyl isostearate and jojoba oil.

The ester oil may be of one type or may be a combination of two or more types. For example, if an ester oil in which diisopropyl sebacate (IOB = 0.4) and cetyl 2-ethylhexanoate (IOB = 0.13) at a ratio of 1 : 1 is used, then the IOB value of the ester oil would be $[0.4 \times 1/2] + [0.13 \times 1/2] = 0.265$, thus fulfilling the conditions of the present invention.

The "liquid oil" of ingredient (d) may, in addition to the ester oil, contain other liquid oil ingredients that are liquid at ambient temperature and that can be blended into cosmetics, such as, for example, hydrocarbon oils, higher fatty acids, higher alcohols and silicone oils.

Examples of hydrocarbon oils include liquid paraffin, ozokerite, squalane, pristane, paraffin, ceresin, squalene, vaselin, microcrystalline wax, polyethylene wax, Fischer-Tropsch waxes and the like.

Examples of higher fatty acids include lauric acid, myristic acid, palmitic acid, stearic acid, behenic acid, arachidonic acid and the like.

Examples of higher alcohols include lauryl alcohol, myristyl alcohol, cetyl alcohol, stearyl alcohol, behenyl alcohol, arachyl alcohol, batyl alcohol, chimyl alcohol, carnaubyl alcohol, ceryl alcohol, corianyl alcohol, myricyl alcohol, lacceryl alcohol, elaidyl alcohol, isostearyl glyceryl ether, octyl alcohol, triacontyl alcohol, selachyl alcohol, cetostearyl alcohol, oleyl alcohol, lanolin alcohol, hydrogenated lanolin alcohol, hexyl decanol, octyl decanol and the like.

Examples of silicone oils include methyl polysiloxane, octamethylsiloxane, decamethyltetrasiloxane, methyl hydrogen polysiloxane, methyl phenyl polysiloxane, hexamethylcyclotrisiloxane, octamethylcyclotetrasiloxane, decamethylcyclopentasiloxane and the like. Preferable examples include octamethylcyclotetrasiloxane, decamethylcyclopentasiloxane and the like.

However, the proportion of the ester oil in the liquid oil (ingredient (d)) should preferably be 30 mass% or more, and more preferably 50 mass% or more. If the proportion of the ester oil is less than 30 mass%, there is a tendency for the photodegradation prevention effect of the ultraviolet ray absorbing agent to become lower.

The blended amount of the liquid oil (ingredient (d)) in the sunscreen cosmetic of the present invention should be 1 to 80 mass%, preferably 3 to 70 mass%, and more preferably 5 to 60 mass%. Therefore, the blended content of the ester oil in the sunscreen cosmetic of the present invention should be 0.3 to 40 mass%, preferably 0.9 to 35 mass%, and more preferably 1.5 to 30 mass%.

The present inventors surmise that interactions between 4-tert-butyl-4'-methoxy dibenzoylmethane and ethylhexyl methoxycinnamate are involved in the photodegradation of the ultraviolet ray protection effect that occurs when (a) 4-tert-butyl-4'-methoxy dibenzoylmethane and (b) ethylhexyl methoxycinnamate coexist. For example, one factor may be that the amount of solvent is reduced by evaporation of water or the like contained in the system, raising the frequency by which 4-tert-butyl-4'-methoxy dibenzoylmethane and ethylhexyl methoxycinnamate approach and collide with each other, and causing the photodegradation to progress due to the interactions therebetween. In the cosmetic of the present invention, the non-volatile (c) amphiphilic substance and (d) liquid oil function as solvents for the ultraviolet ray absorbing agents so that, even after water and the like evaporate, the frequency of the interactions between the 4-tert-butyl-4'-methoxy dibenzoylmethane and the ethylhexyl methoxycinnamate is limited, thereby preventing photodegradation.

Therefore, the present invention provides a method for preventing photodegradation of a sunscreen cosmetic, comprising a step of blending, into a sunscreen cosmetic containing (a) 4-tert-butyl-4'-methoxy dibenzoylmethane and (b) ethylhexyl methoxycinnamate, (c) 5.0 mass% or more of an amphiphilic substance and (d) a liquid oil containing an ester oil having an IOB of 0.05 to 0.60.

In the sunscreen cosmetic of the present invention, it is possible to blend the maximum possible amounts of (a) 4-tert-butyl-4'-methoxy dibenzoylmethane, which is a UVA absorbing agent, and (b) ethylhexyl methoxycinnamate, which is a UVB absorbing agent, and the photodegradation of the ultraviolet ray absorbing agents is suppressed by blending in the liquid oil containing (c) the amphiphilic substance and (d) the liquid oil containing an ester oil having an IOB of 0.05 to 0.60. Thus, sufficiently high ultraviolet ray protection performance (for example, an SPF of 15 or more) is achieved for a long period of time across a wide wavelength range spanning from the UVA to the UVB ranges.

However, the ultraviolet ray protection effects in the UVA and/or the UVB ranges can be further improved by further blending in other ultraviolet ray absorbing agents aside from (a) 4-tert-butyl-4'-methoxy dibenzoylmethane and (b) ethylhexyl methoxycinnamate. Alternatively, the cosmetic may be formulated by reducing the blended amounts of 4-tert-butyl-4'-methoxy dibenzoylmethane and/or ethylhexyl methoxycinnamate.

The other ultraviolet ray absorbing agents may be chosen from those that are normally used in cosmetics, and are not particularly limited. Examples include one or more substances chosen from among para-aminobenzoic acid derivatives, salicylic acid derivatives, β,β-diphenyl acrylate derivatives, benzophenone derivatives, benzylidene camphor derivatives, phenyl benzimidazole derivatives, triazine derivatives, phenyl benzotriazole derivatives, anthranil derivatives, imidazoline derivatives, benzalmalonate derivatives, 4,4-diaryl butadiene derivatives and the like.

The sunscreen cosmetic of the present invention may, in addition to the above-mentioned ultraviolet ray absorbing agents, contain a powder (ultraviolet ray scattering agent) that physically screens ultraviolet rays by reflecting or scattering them.

The ultraviolet ray scattering agent that is used in the present invention is not particularly limited as long as it is a powder that is used as an ultraviolet ray scattering agent in the field of cosmetics. Specific examples include one or more substances chosen from among titanium oxide, zinc oxide, barium sulfate, iron oxide, talc, mica, sericite, kaolin, titanated mica, Prussian blue, chromium oxide, chromium hydroxide, silica, cerium oxide and the like. In particular, it is preferable, in terms of optical properties, to use a powder having a refractive index of at least 1.5 such as, for example, zinc oxide or titanium oxide.

The dispersibility in oil and the water resistance of an ultraviolet ray scattering agent can be improved by surface hydrophobization, and surface-hydrophobized ultraviolet ray scattering agents may also be favorably used in the present invention.

Examples of the surface treatment method include silicone treatments using methyl hydrogen polysiloxane, methyl polysiloxane or the like; alkyl silane treatments; fluorine treatments using perfluoroalkyl phosphate esters, perfluoroalcohols or the like; amino acid treaments using N-acyl glutamic acid or the like; and aside therefrom, lecithin treatments; metal soap treatments; fatty acid treatments; and alkyl phosphate ester treatments.

The ultraviolet ray scattering agent used in the present invention is not particularly limited, but should normally have an average primary particle size of 100 nm or less, more preferably 80 nm or less. If the average primary particle size greatly exceeds 100 nm, then a tendency to cause unnatural whiteness or residual whiteness is observed.

In the present specification, the average primary particle size is a value that is determined, for example, as the arithmetic mean of the lengths of the long axes and the lengths of the short axes of the particles based on transmission electron microscope photographs.

Additionally, the shapes of the particles in the ultraviolet ray scattering agent are not particularly limited, and the particles may be in the form of primary particles, or may form aggregated secondary clusters. Furthermore, they are not particularly limited as to the shape, such as whether they are spherical, elliptical, crushed or the like.

When blending an ultraviolet ray scattering agent into the sunscreen cosmetic of the present invention, the blended amount thereof should be 1 to 30 mass%, preferably 3 to 25 mass%. If the blended amount is less than 1 mass%, then the resulting improvement in the ultraviolet ray protection effects is not sufficient, and if more than 30 mass% is blended, then the whiteness becomes noticeable at the time of application, and there may be a powdery sensation when used. Since the sunscreen cosmetic of the present invention obtains sufficient ultraviolet ray protection effects due to the ultraviolet ray absorbing agents, it may be provided in a form not containing an ultraviolet ray scattering agent.

In addition to the aforementioned essential ingredients and other ultraviolet ray absorbing agents and ultraviolet ray scattering agents, the sunscreen cosmetic of the present invention may contain other optional ingredients that can normally be blended into sunscreen cosmetics, within a range not inhibiting the effects of the present invention.

Examples of the other optional ingredients include, but are not limited to, water-soluble polymers, oil-soluble polymers, solid oils such as waxes, lower alcohols, humectant ingredients, surfactants, powder ingredients and pharmaceutical agents.

Examples of water-soluble polymers include homopolymers or copolymers of 2-acrylamido-2-methylpropane sulfonic acid (hereinafter abbreviated to "AMPS"). The copolymers are copolymers comprising comonomers such as vinyl pyrrolidone, acrylic acid amides, sodium acrylate and hydroxyethyl acrylate. In other words, examples include AMPS homopolymers, vinyl pyrrolidone/AMPS copolymers, dimethyl acrylamide/AMPS copolymers, acrylic acid amide/AMPS copolymers, sodium acrylate/AMPS copolymers and the like.

Further examples include carboxyvinyl polymers, ammonium polyacrylate, sodium polyacrylates, sodium acrylate/alkyl acrylate/sodium methacrylate/alkyl methacrylate copolymers, carrageenan, pectin, mannan, curdlan, chondroitin sulfate, starch, glycogen, gum arabic, sodium hyaluronate, tragacanth gum, xanthan gum, mucoitin sulfate, hydroxyethyl guar gum, carboxymethyl guar gum, guar gum, dextran, keratosulfate, locust bean gum, succcinoglucan, chitin, chitosan, carboxymethyl chitin, agar and the like.

Examples of the oil-soluble polymer include trimethylsiloxysilicate, alkyl-modified silicone, polyamide-modified silicone, dimethicone cross-polymers, (dimethicone/vinyl dimethicone) cross-polymers, poylmethylsilsesquioxane and the like.

Examples of waxes include beeswax, candelilla wax, carnauba wax, lanolin, liquid lanolin, jojoba wax and the like.

Examples of lower alcohols include alcohols having 1 to 5 carbon atoms, such as ethanol and isopropanol.

Examples of humectant ingredients include polyhydric alcohols such as ethylene glycol, propylene glycol, 1,3-butylene glycol, dipropylene glycol and polybutylene glycol.

Examples of surfactants include anionic, cationic, non-ionic or amphoteric surfactants, including silicone-based or hydrocarbon-based surfactants.

Examples of powder ingredients include nylon or acrylic polymer spherical powders, silica powders, silicone powders, metal oxide powders that have been surface-treated with surface treating agents not containing metals, and the like.

Examples of pharmaceutical agents include L-ascorbic acid and derivative salts thereof, glycyrrhizic acid and derivatives thereof such as dipotassium glycyrrhizate and monoammonium glycyrrhizate, glycyrrhetinic acid and derivatives thereof such as stearyl glycyrrhetinate, allantoin, tranexamic acid and derivative salts thereof, alkoxysalicylic acid and derivative salts thereof, glutathione and derivative salts thereof, allantoin, azulene and the like.

Examples of other ingredients include sugars such as trehalose and erythritol; polysaccharides such as hyaluronic acid and acetylated hyaluronic acid; amino acids such as serine, glycine, hydroxyproline, glutamic acid and alanine; and extracts such as silk extract, *Chlorella* extract, hydrolyzed pearl protein, *Rodgersia podophylla* extract, *Rubus suavissimus* extract, yeast extract, inositol, *Glycyrrhiza glabra* extract, *Sanguisorba officinalis* extract, rose extract and coix seed extract.

The sunscreen cosmetic of the present invention may be provided in the form of an oil-in-water emulsified cosmetic, a water-in-oil emulsified cosmetic or an oil-based cosmetic. Specific formats include formats such as sunscreen lotions and sunscreen creams, which may be manufactured using conventional methods that are appropriate for each format.

EXAMPLES

Herebelow, the present invention will be described in further detail by giving examples, but the present invention is not to be construed as being limited thereto. Where not specifically noted, blended amounts are indicated in mass% with respect to the system in which the ingredient is contained.

(1) Water-in-oil emulsified sunscreen cosmetic

(1-1) Samples of water-in-oil emulsified sunscreen cosmetics were prepared with the compositions shown in Tables 1 and 2 below. Specifically, the oil-based ingredients were mixed, a clay mineral (dimethyl distearyl ammonium hectorite) was dispersed therein, the separately mixed water-based ingredients were added, and the resulting mixture was emulsified with a homomixer.

Next, samples of each example were evaluated for the photostability of the sunscreen cosmetic (stability of ultraviolet absorption performance) by the following method. The photostability was judged to be high if the retention rate was 60% or more, or if the ratio of the retention rate relative to a reference level (improvement rate) exceeded 103%.

A sample of each example was applied to a PMMA substrate and the absorbance in the range of 500 to 280 nm was measured. After irradiating the substrate with UV rays for 2 hours using a Suntest testing machine, the absorbance was measured in the same manner, and the change in the integrated absorbance value in the aforementioned wavelength range before and after irradiation (post-photoirradiation ultraviolet ray absorption performance retention rate) was calculated in accordance with the following formula:

Post-photoirradiation retention rate (%) = (post-photoirradiation integrated absorbance value)/(pre-photoirradiation integrated absorbance value) $\times$ 100

The values of the post-photoirradiation retention rate (%) for each example are also shown in Table 2.

[Table 1]

| Raw Material | Blended Amount (mass%) |
|---|---|
| Purified water | bal |
| Ethanol | 3 |
| PEG-9 polydimethylsiloxyethyl dimethicone | 0.8 |
| Dimethyl distearyl ammonium hectorite | 0.2 |
| Cyclomethicone | 5 |
| 2-ethylhexyl para-methoxycinnamate | 7.5 |
| 4-tert-butyl-4'-methoxy dibenzoylmethane | 2.5 |
| Triethylhexanoin (IOB = 0.36) | 30 |
| Isododecane | 24 |
| Amphiphilic substance (Table 2) | Table 2 (*) |
| Total | 100 |

[Table 2]

| | Amphiphilic Substance | Blended Amount (*) | Post-photo-irradiation Retention Rate (%) |
|---|---|---|---|
| Example 1 | Polyoxyethylene(14) polyoxypropylene(7) dimethyl ether | 10 | 70 |
| Example 2 | Polyoxyethylene(3) polyoxypropylene(6) dimethyl ether | 10 | 76 |
| Example 3 | Polyoxyethylene(6) polyoxypropylene(3) dimethyl ether | 10 | 70 |
| Example 4 | Polyoxyethylene(8) polyoxypropylene(4) dimethyl ether | 10 | 72 |
| Example 5 | Polyoxyethylene(9) polyoxypropylene(2) dimethyl ether | 10 | 90 |
| Example 6 | Polyoxyethylene(11) polyoxypropylene(6) dimethyl ether | 10 | 73 |
| Example 7 | Polyoxyethylene(17) polyoxypropylene(4) dimethyl ether | 10 | 74 |
| Example 8 | Polyoxyethylene(27) polyoxypropylene(14) dimethyl ether | 10 | 73 |
| Example 9 | Polyoxyethylene(36) polyoxypropylene(41) dimethyl ether | 10 | 73 |
| Example 10 | Polyoxyethylene(55) polyoxypropylene(28) dimethyl ether | 10 | 82 |
| Example 11 | Polyethylene glycol 400 | 5 | 62 |
| Example 12 | Glycerin | 5 | 60 |

(continued)

| | Amphiphilic Substance | Blended Amount (*) | Post-photo-irradiation Retention Rate (%) |
|---|---|---|---|
| Example 13 | Dipropylene glycol | 5 | 66 |
| Example 14 | Polypropylene glycol 1000 | 5 | 74 |
| Example 15 | Polyoxybutylene(9) polyoxypropylene(1) glycol | 5 | 76 |
| Example 16 | Polyoxyethylene(8) polyoxypropylene(4) dimethyl ether | 5 | 70 |
| Example 17 | Polyoxyethylene(36) polyoxypropylene(41) dimethyl ether | 5 | 72 |
| Example 18 | Polyoxyethylene(11) polyoxypropylene(6) dimethyl ether | 5 | 73 |
| Comparative Example 1 | None | 0 | 54 |

As is clear from the results shown in Table 2, in Examples 1 to 18 containing ester oils having an IOB of 0.05 to 0.60 and 5 mass% or more of an amphiphilic substance, the post-photoirradiation retention rate significantly increased relative to the case in which no amphiphilic substance was contained (Comparative Example 1)

(1-2) Evaluations similar to those in (1-1) above were performed on the water-in-oil emulsified sunscreen cosmetics having the compositions shown in the following Tables 3 to 5. The evaluation results are also shown in Tables 3 to 5.

[Table 3]

| Raw Material | Ex. 19 | Ex. 20 | Ex. 21 | Ex. 22 | Ex. 23 | Comp. Ex. 2 |
|---|---|---|---|---|---|---|
| Purified water | bal | bal | bal | bal | bal | bal |
| Lauryl PEG-9 polydimethylsiloxyethyl dimethicone | 0.8 | 0.8 | 0.8 | 0.8 | 0.8 | 0.8 |
| Dimethyl distearyl ammonium hectorite | 0.4 | 0.4 | 0.4 | 0.4 | 0.4 | 0.4 |
| Cyclomethicone | 5 | 5 | 5 | 5 | 5 | 5 |
| 2-Ethylhexyl para-methoxycinnamate | 7.5 | 7.5 | 7.5 | 7.5 | 7.5 | 7.5 |
| 4-Tert-butyl-4'-methoxy dibenzoylmethane | 2.5 | 2.5 | 2.5 | 2.5 | 2.5 | 2.5 |
| Tripropylene glycol pivalate (IOB = 0.52) | 30 | - | - | - | - | - |
| Diisopropyl sebacate (IOB = 0.40) | - | 30 | - | - | - | - |
| Triethylhexanoin (IOB = 0.36) | - | - | 30 | - | - | - |
| Pentaerythritol tetra-2-ethylhexanoate (IOB = 0.35) | - | - | - | 30 | - | - |
| Cetyl 2-ethylhexanoate (IOB = 0.13) | - | - | - | - | 30 | - |
| Dimethicone | - | - | - | - | - | 30 |
| Isostearic acid | 0.2 | 0.2 | 0.2 | 0.2 | 0.2 | 0.2 |
| Talc | 10 | 10 | 10 | 10 | 10 | 10 |
| Glycerin | 3 | 3 | 3 | 3 | 3 | 3 |
| Isododecane | 24 | 24 | 24 | 24 | 24 | 24 |
| Ethanol | 3 | 3 | 3 | 3 | 3 | 3 |
| Polyoxyethylene(55) polyoxypropylene(28) dimethyl ether | 10 | 10 | 10 | 10 | 10 | 10 |
| Trisodium edetate | s.a. | s.a. | s.a. | s.a. | s.a. | s.a. |
| Table salt | s.a. | s.a. | s.a. | s.a. | s.a. | s.a. |

(continued)

| Raw Material | Ex. 19 | Ex. 20 | Ex. 21 | Ex. 22 | Ex. 23 | Comp. Ex. 2 |
|---|---|---|---|---|---|---|
| Total | 100 | 100 | 100 | 100 | 100 | 100 |
| Post-photoirradiation retention rate (%) | 74.6 | 73.5 | 76.7 | 80.5 | 81.5 | 49.2 |

[Table 4]

| Raw Material | Ex. 24 | Ex. 25 | Ex. 26 | Ex. 27 | Ex. 28 | Ex. 29 | Ex. 30 |
|---|---|---|---|---|---|---|---|
| Purified water | bal | bal | bal | bal | bal | bal | bal |
| Lauryl PEG-9 polydimethylsiloxyethyl dimethicone | 0.8 | 0.8 | 0.8 | 0.8 | 0.8 | 0.8 | 0.8 |
| Dimethyl distearyl ammonium hectorite | 0.4 | 0.4 | 0.4 | 0.4 | 0.4 | 0.4 | 0.4 |
| Cyclomethicone | 5 | 5 | 5 | 5 | 5 | 5 | 5 |
| 2-Ethylhexyl para-methoxycinnamate | 7.5 | 7.5 | 7.5 | 7.5 | 7.5 | 7.5 | 7.5 |
| 4-Tert-butyl-4'-methoxy dibenzoylmethane | 2.5 | 2.5 | 2.5 | 2.5 | 2.5 | 2.5 | 2.5 |
| Diisopropyl sebacate (IOB = 0.40) | - | - | - | - | - | - | - |
| Isopropyl myristate (IOB = 0.18) | - | - | - | 10 | 10 | 5 | |
| Triethylhexanoin (IOB = 0.36) | 10 | 5 | 5 | - | - | - | - |
| Cetyl 2-ethylhexanoate (IOB = 0.13) | 10 | 10 | 5 | 10 | 5 | 10 | - |
| Mineral oil | 10 | 15 | 20 | - | - | - | - |
| $\alpha$-Olefin oligomer | - | - | - | 15 | | | |
| Dimethicone | 2 | 2 | 2 | 2 | 2 | 2 | 2 |
| Polyoxybutylene(9) polyoxypropylene(1) glycol | 2 | 2 | 2 | 2 | 2 | 2 | 2 |
| Isostearic acid | 0.2 | 0.2 | 0.2 | 0.2 | 0.2 | 0.2 | 0.2 |
| Talc | 10 | 10 | 10 | 10 | 10 | 10 | 10 |
| Glycerin | 3 | 3 | 3 | 3 | 3 | 3 | 3 |
| Isododecane | 24 | 24 | 24 | 24 | 24 | 24 | 24 |
| Ethanol | 3 | 3 | 3 | 3 | 3 | 3 | 3 |
| Polyoxyethylene(55) polyoxypropylene(28) dimethyl ether | 10 | 10 | 10 | 10 | 10 | 10 | 10 |
| Trisodium edetate | s.a. | s.a. | s.a. | s.a. | s.a. | s.a. | s.a. |
| Table salt | s.a. | s.a. | s.a. | s.a. | s.a. | s.a. | s.a. |
| Total | 100 | 100 | 100 | 100 | 100 | 100 | 100 |
| IOB of ester oil | 0.25 | 0.21 | 0.25 | 0.16 | 0.16 | 0.15 | 0.16 |
| Post-photoirradiation retention rate (%) | 73.8 | 74.1 | 67.9 | 75.1 | 73.7 | 74.7 | 66.5 |

[Table 5]

| Raw Material | Ex. 31 | Ex. 32 | Ex. 33 | Ex. 34 | Ex. 35 | Ex. 36 | Comp. Ex. 3 |
|---|---|---|---|---|---|---|---|
| Purified water | bal | bal | bal | bal | bal | bal | bal |
| Lauryl PEG-9 polydimethylsiloxyethyl dimethicone | 0.8 | 0.8 | 0.8 | 0.8 | 0.8 | 0.8 | 0.8 |
| Dimethyl distearyl ammonium hectorite | 0.4 | 0.4 | 0.4 | 0.4 | 0.4 | 0.4 | 0.4 |

(continued)

| Raw Material | Ex. 31 | Ex. 32 | Ex. 33 | Ex. 34 | Ex. 35 | Ex. 36 | Comp. Ex. 3 |
|---|---|---|---|---|---|---|---|
| Cyclomethicone | 5 | 5 | 5 | 5 | 5 | 5 | 5 |
| 2-Ethylhexyl para-methoxycinnamate | 7.5 | 7.5 | 7.5 | 7.5 | 7.5 | 7.5 | 7.5 |
| 4-Tert-butyl-4'-methoxy dibenzoylmethane | 2.5 | 2.5 | 2.5 | 2.5 | 2.5 | 2.5 | 2.5 |
| Diisopropyl sebacate (IOB = 0.40) | 10 | 10 | 5 | 5 | 4 | 3 | - |
| Isopropyl myristate (IOB = 0.18) | - | - | - | - | - | - | - |
| Triethylhexanoin (IOB = 0.36) | - | - | - | - | - | - | - |
| Cetyl 2-ethylhexanoate (IOB = 0.13) | 10 | 5 | 10 | 5 | 4 | 3 | - |
| Mineral oil | - | - | - | - | - | - | - |
| $\alpha$-Olefin oligomer | 10 | 15 | 15 | 20 | 16 | 12 | 30 |
| Dimethicone | 2 | 2 | 2 | 2 | 2 | 2 | 2 |
| Polyoxybutylene(9) polyoxypropylene(1) glycol | 2 | 2 | 2 | 2 | 2 | 2 | 2 |
| Isostearic acid | 0.2 | 0.2 | 0.2 | 0.2 | 0.2 | 0.2 | 0.2 |
| Talc | 10 | 10 | 10 | 10 | 10 | 10 | 10 |
| Glycerin | 3 | 3 | 3 | 3 | 3 | 3 | 3 |
| Isododecane | 24 | 24 | 24 | 24 | 24 | 24 | 24 |
| Ethanol | 3 | 3 | 3 | 3 | 3 | 3 | 3 |
| Polyoxyethylene(55) polyoxypropylene(28) dimethyl ether | 10 | 10 | 10 | 10 | 10 | 10 | 10 |
| Trisodium edetate | s.a. | s.a. | s.a. | s.a. | s.a. | s.a. | s.a. |
| Table salt | s.a. | s.a. | s.a. | s.a. | s.a. | s.a. | s.a. |
| Total | 100 | 100 | 100 | 100 | 100 | 100 | 100 |
| IOB of ester oil | 0.26 | 0.31 | 0.22 | 0.27 | 0.27 | 0.27 | - |
| Post-photoirradiation retention rate (%) | 76.1 | 76.2 | 72.4 | 70.4 | 69.1 | 68.4 | 55.9 |

As is clear from the results shown in Tables 3 to 5, as long as the IOB value of the blended ester oil was within the range specified in the present invention, excellent photostabilization effects were obtained irrespective of the type and blended amount of the ester oil.

(1-3) Evaluations similar to those in (1-1) above were performed on the water-in-oil emulsified sunscreen cosmetics having the compositions shown in the following Table 6. The evaluation results are also shown in Table 6.

[Table 6]

| Raw Material | Comp. Ex. 4 | Comp. Ex. 5 | Comp. Ex. 6 | Ex. 37 | Ex. 38 | Ex. 39 |
|---|---|---|---|---|---|---|
| Purified water | | | | | | |
| Lauryl PEG-9 polydimethylsiloxyethyl dimethicone | 0.8 | 0.8 | 0.8 | 0.8 | 0.8 | 0.8 |
| 2-Ethylhexyl para-methoxycinnamate | 7.5 | 7.5 | 7.5 | 7.5 | 7.5 | 7.5 |
| 4-Tert-butyl-4'-methoxy dibenzoylmethane | 2.5 | 2.5 | 2.5 | 2.5 | 2.5 | 2.5 |
| Diisopropyl sebacate (IOB = 0.40) | 10 | 10 | 10 | 10 | 10 | 10 |

(continued)

| Raw Material | Comp. Ex. 4 | Comp. Ex. 5 | Comp. Ex. 6 | Ex. 37 | Ex. 38 | Ex. 39 |
|---|---|---|---|---|---|---|
| Triethylhexanoin (IOB = 0.36) | 3 | 3 | 3 | 3 | 3 | 3 |
| Dimethicone | 2 | 2 | 2 | 2 | 2 | 2 |
| Isododecane | 15 | 15 | 15 | 15 | 15 | 15 |
| Isostearic acid | 0.5 | 0.5 | 0.5 | 0.5 | 0.5 | 0.5 |
| Crosslinked silicone/reticulated silicone block copolymer | 3 | 3 | 3 | 3 | 3 | 3 |
| Methylsiloxane reticulated polymer | 3 | 3 | 3 | 3 | 3 | 3 |
| Glycerin | 2 | 2 | 2 | 2 | 2 | 2 |
| Ethanol | 3 | 3 | 3 | 3 | 3 | 3 |
| Polyoxyethylene(9) polyoxypropylene(2) dimethyl ether | - | 1 | 3 | 5 | 8 | 10 |
| Trisodium edetate | s.a. | s.a. | s.a. | s.a. | s.a. | s.a. |
| Table salt | s.a. | s.a. | s.a. | s.a. | s.a. | s.a. |
| IOB of ester oil | 0.39 | 0.39 | 0.39 | 0.39 | 0.39 | 0.39 |
| Post-photoirradiation retention rate (%) (improvement rate %) | 54.5 (ref) | 50.7 | 49.8 | 57.3 (105.3) | 65.8 | 63.6 |

From the results shown in Table 6, it was confirmed that sufficient photostabilization effects cannot be obtained even when an ester oil of the prescribed IOB value and an amphiphilic substance are blended, unless the blended amount of the amphiphilic substance is at least 5 mass%.

(1-4) Evaluations similar to those in (1-1) above were performed on the water-in-oil emulsified sunscreen cosmetics having the compositions shown in the following Table 7. The evaluation results are also shown in Table 7.

[Table 7]

| Raw Material | Ex. 40 | Ex. 41 | Ex. 42 | Ex. 43 | Ex. 44 |
|---|---|---|---|---|---|
| Purified water | bal | bal | bal | bal | bal |
| Lauryl PEG-9 polydimethylsiloxyethyl dimethicone | 0.8 | 0.8 | 0.8 | 0.8 | 0.8 |
| 2-Ethylhexyl para-methoxycinnamate | 7.5 | 7.5 | 7.5 | 7.5 | 7.5 |
| 4-Tert-butyl-4'-methoxy dibenzoylmethane | 2.5 | 2.5 | 2.5 | 2.5 | 2.5 |
| Octocrylene | 2 | 5 | - | - | - |
| 2,4-Bis-[{4-(2-ethylhexyloxy)-2-hydroxy}-phenyl]-6-(4-methoxyphenyl)-1,3,5-triazine | - | - | 1 | 2 | - |
| Diethylaminohydroxybenzoyl hexyl benzoate | - | - | - | - | 2 |
| Diisopropyl sebacate (IOB = 0.40) | 10 | 10 | 10 | 10 | 10 |
| Triethylhexanoin (IOB = 0.36) | 3 | 3 | 3 | 3 | 3 |
| Dimethicone | 2 | 2 | 2 | 2 | 2 |
| Isododecane | 15 | 15 | 15 | 15 | 15 |
| Isostearic acid | 0.5 | 0.5 | 0.5 | 0.5 | 0.5 |
| Crosslinked silicone/reticulated silicone block copolymer | 3 | 3 | 3 | 3 | 3 |
| Methylsiloxane reticulated polymer | 3 | 3 | 3 | 3 | 3 |

(continued)

| Raw Material | Ex. 40 | Ex. 41 | Ex. 42 | Ex. 43 | Ex. 44 |
|---|---|---|---|---|---|
| Glycerin | 2 | 2 | 2 | 2 | 2 |
| Ethanol | 3 | 3 | 3 | 3 | 3 |
| Polyoxyethylene(55) polyoxypropylene(28) dimethyl ether | 10 | 10 | 10 | 10 | 10 |
| Trisodium edetate | s.a. | s.a. | s.a. | s.a. | s.a. |
| Table salt | s.a. | s.a. | s.a. | s.a. | s.a. |
| IOB of ester oil | 0.39 | 0.39 | 0.39 | 0.39 | 0.39 |
| Post-photoirradiation retention rate (%) | 71.3 | 76.4 | 81.3 | 75.1 | 83.5 |

From the results shown in Table 7, it was confirmed that sufficient photostabilization effects can be obtained in a cosmetic in which an ester oil of the prescribed IOB value and an amphiphilic substance are blended, and in which other ultraviolet ray absorbing agents are further blended.

(2) Oil-in-water emulsified sunscreen cosmetic

Samples of oil-in-water emulsified sunscreen cosmetics were prepared with the compositions shown in Tables 8 and 9 below. Specifically, a thickener (a carbomer or the like) was dissolved into the water-based ingredients, the separately mixed and dissolved oil-based ingredients were added thereto, and the resulting mixture was emulsified with a homomixer.

Evaluations similar to those in (1-1) above were performed on the cosmetics of the resulting examples. The evaluation results are also shown in Tables 8 and 9.

[Table 8]

| Raw Material | Comp. Ex. 7 | Ex. 45 | Ex. 46 | Ex. 47 | Ex. 48 |
|---|---|---|---|---|---|
| Purified water | bal | bal | bal | bal | bal |
| Acrylic acid/alkyl methacrylate copolymer | 0.2 | 0.2 | 0.2 | 0.2 | 0.2 |
| Carbomer | 0.4 | 0.4 | 0.4 | 0.4 | 0.4 |
| Potassium hydroxide | s.a. | s.a. | s.a. | s.a. | s.a. |
| 2-Ethylhexyl para-methoxycinnamate | 8 | 8 | 8 | 8 | 8 |
| 4-Tert-butyl-4'-methoxy dibenzoylmethane | 2 | 2 | 2 | 2 | 2 |
| Cetyl 2-ethylhexanoate (IOB = 0.13) | 10 | 10 | 10 | 10 | 10 |
| Glycerin | 5 | 5 | 5 | 5 | 5 |
| Dipropylene glycol | 7 | 7 | 7 | 7 | 7 |
| Polyoxyethylene(14) polyoxypropylene(7) dimethyl ether | - | 5 | 10 | - | - |
| Polyoxyethylene(9) polyoxypropylene(2) dimethyl ether | - | - | - | 10 | - |
| Polyoxyethylene(55) polyoxypropylene(28) dimethyl ether | - | - | - | - | 10 |
| Post-photoirradiation retention rate (%) (improvement rate %) | 53 (ref) | 66 | 74 | 61 | 57 (107.5) |

[Table 9]

| Raw Material | Ex. 49 | Ex. 50 | Ex. 51 | Ex. 52 |
|---|---|---|---|---|
| Purified water | bal | bal | bal | bal |
| Polyoxyethylene(20) polyoxypropylene(8) cetyl ether | 3 | 3 | 3 | 3 |
| Glycerin | 5 | 5 | 5 | 5 |

(continued)

| Raw Material | Ex. 49 | Ex. 50 | Ex. 51 | Ex. 52 |
|---|---|---|---|---|
| Dipropylene glycol | 7 | 7 | 7 | 7 |
| Cetyl 2-ethylhexanoate (IOB = 0.13) | 10 | 10 | 10 | 10 |
| 2-Ethylhexyl para-methoxcinnamate | 8 | 8 | 8 | 8 |
| 4-Tert-butyl-4'-methoxy dibenzoylmethane | 2 | 2 | 2 | 2 |
| Carbomer | 0.1 | 0.1 | 0.1 | 0.1 |
| Potassium hydroxide | s.a. | s.a. | s.a. | s.a. |
| Polyoxyethylene(14) polyoxypropylene(7) dimethyl ether | 5 | 10 | - | - |
| Polyoxyethylene(9) polyoxypropylene(2) dimethyl ether | - | - | 10 | - |
| Polyoxyethylene(55) polyoxypropylene(28) dimethyl ether | - | - | - | 10 |
| Post-photoirradiation retention rate (%) | 80 | 84 | 91 | 73 |

From the results shown in Table 8 and 9, it was confirmed that excellent photostabilization effects can be obtained by blending an ester oil having the prescribed IOB value and an amphiphilic substance, even in oil-in-water emulsified form.
Herebelow, other formulations (examples) of the sunscreen cosmetic according to the present invention will be described. Excellent photostabilization effects were also obtained for these cosmetics.

Formulation 1: Oil-in-water sunscreen gel

[Table 10]

| Raw Material | Ex. 53 |
|---|---|
| Purified water | bal |
| Polyoxyethylene(20) polyoxypropylene(8) cetyl ether | 3 |
| Glycerin | 5 |
| Dipropylene glycol | 4 |
| Cetyl 2-ethylhexanoate (IOB = 0.13) | 10 |
| 2-Ethylhexyl para-methoxcinnamate | 8 |
| 4-Tert-butyl-4'-methoxy dibenzoylmethane | 2 |
| Carbomer | 0.1 |
| Potassium hydroxide | s.a. |
| Polyoxyethylene(14) polyoxypropylene(7) dimethyl ether | 5 |
| Fragrance | s.a. |
| Hyaluronic acid | 0.1 |
| Rose extract | 0.2 |
| Dipotassium glycyrrhizate | 0.5 |
| 1,3-Butylene glycol | 3 |
| Total | 100 |

Production method: A thickener was dissolved into the water-based ingredients, the separately mixed and dissolved oil-based ingredients were added thereto, and the resulting mixture was emulsified with a homomixer.

Formulation 2: Oil-in-water whitening protector

[Table 11]

| Raw Material | Ex. 54 |
|---|---|
| Purified water | bal |
| Glycerin | 5 |
| Dipropylene glycol | 4 |
| Mineral oil | 10 |
| Isopropyl myristate | 5 |
| 2-Ethylhexyl para-methoxycinnamate | 6 |
| 4-Tert-butyl-4'-methoxy dibenzoylmethane | 3 |
| Carbomer | 0.2 |
| Acrylic acid/alkyl methacrylate copolymer | 0.1 |
| Potassium hydroxide | s.a. |
| Polyoxyethylene(9) polyoxypropylene(11) dimethyl ether | 5 |
| Fragrance | s.a. |
| Tranexamic acid | 2.5 |
| Alkoxysalicylic acid | 1 |
| Acetylated hyaluronic acid | 0.1 |
| *Sanguisorba officinalis* extract | 0.2 |
| Dipotassium glycyrrhizate | 0.5 |
| 1,3-Butylene glycol | 3 |
| Total | 100 |

Production method: A thickener was dissolved into the water-based ingredients, the separately mixed and dissolved oil-based ingredients were added thereto, and the resulting mixture was emulsified with a homomixer.

Formulation 3: Water-in-oil sunscreen emulsion

[Table 12]

| Raw Material | Ex. 55 |
|---|---|
| Purified water | bal |
| Lauryl PEG-9 polydimethylsiloxyethyl dimethicone | 0.8 |
| 2-Ethylhexyl para-methoxycinnamate | 7.5 |
| 4-Tert-butyl-4'-methoxy dibenzoylmethane | 2.5 |
| Octocrylene | 2 |
| 2,4-Bis-[{4-(2-ethylhexyloxy)-2-hydroxy}-phenyl]-6-(4-methoxyphenyl)-1,3,5-triazine | 1 |
| Diethylaminohydroxybenzoyl hexyl benzoate | 0.3 |
| Diisopropyl sebacate | 10 |
| Triethylhexanoin | 3 |

(continued)

| Raw Material | Ex. 55 |
|---|---|
| Dimethicone | 2 |
| Isododecane | 15 |
| Isostearic acid | 0.5 |
| Crosslinked silicone/reticulated silicone block copolymer | 3 |
| Methylsiloxane reticulated polymer | 3 |
| Inositol | 2 |
| Ethanol | 3 |
| Polyoxyethylene(55) polyoxypropylene(28) dimethyl ether | 10 |
| Zinc oxide | 5 |
| Titanium oxide | 3 |
| Vitamin C ethyl | 0.5 |
| Glutamic acid | 0.1 |
| Trisodium edetate | s.a. |
| Table salt | s.a. |
| Total | 100 |

Production method: The oil-based ingredients were mixed, the powders were dispersed therein, the separately mixed water-based ingredients were added, and the resulting mixture was emulsified with a homomixer.

Formulation 4: Water-in-oil sunscreen spray

[Table 13]

| Raw Material | Ex. 56 |
|---|---|
| Purified water | bal |
| Polyoxyethylene/methyl polysiloxane copolymer | 0.8 |
| 2-Ethylhexyl para-methoxycinnamate | 3 |
| 4-Tert-butyl-4'-methoxy dibenzoylmethane | 1 |
| 2,4-Bis-[{4-(2-ethylhexyloxy)-2-hydroxy}-phenyl]-6-(4-methoxyphenyl)-1,3,5-triazine | 0.5 |
| Diisopropyl sebacate | 5 |
| Triethylhexanoin | 3 |
| Cyclomethicone | 5 |
| Isododecane | 4 |
| Isostearic acid | 0.5 |
| Titanium oxide | 3 |
| Iron oxide | 0.3 |
| Crosslinked silicone/reticulated silicone block copolymer | 5 |
| Xylitol | 2 |
| Ethanol | 4 |

(continued)

| Raw Material | Ex. 56 |
|---|---|
| Polyoxyethylene(7) polyoxypropylene(14) dimethyl ether | 3 |
| Vitamin C ethyl | 0.5 |
| Hyaluronic acid | 0.1 |
| Trisodium edetate | s.a. |
| L.P.G. | 40 |
| Total | 100 |

Production method: The oil-based ingredients were mixed, the powders were dispersed therein, the separately mixed water-based ingredients were added, and the mixture was emulsified with a homomixer. The resulting stock solution and liquefied petroleum gas (LPG) were loaded into an aerosol can at a designated ratio.

Formulation 5: Water-in-oil cosmetic base cream

[Table 14]

| Raw Material | Ex. 57 |
|---|---|
| Purified water | bal |
| Lauryl PEG-9 polydimethylsiloxyethyl dimethicone | 0.8 |
| 2-Ethylhexyl para-methoxycinnamate | 7.5 |
| 4-Tert-butyl-4'-methoxy dibenzoylmethane | 2.5 |
| Octocrylene | 2 |
| 2,4-Bis-[{4-(2-ethylhexyloxy)-2-hydroxy}-phenyl]-6-(4-methoxyphenyl)-1,3,5-triazine | 0.5 |
| Diethylaminohydroxybenzoyl hexyl benzoate | 1 |
| Diisopropyl sebacate | 10 |
| Triethylhexanoin | 3 |
| Dimethicone | 2 |
| Isododecane | 15 |
| Isostearic acid | 1 |
| Crosslinked silicone/reticulated silicone block copolymer | 6 |
| Methylsiloxane reticulated polymer | 4 |
| Titanium oxide | 5 |
| Iron oxide | 1 |
| Inositol | 2 |
| Ethanol | 3 |
| Polyoxyethylene(55) polyoxypropylene(28) dimethyl ether | 5 |
| Vitamin C ethyl | 0.5 |
| Glutamic acid | 0.1 |
| Dimethyl distearyl ammonium hectorite | 3 |
| Trisodium edetate | s.a. |
| Table salt | s.a. |

(continued)

| Raw Material | Ex. 57 |
|---|---|
| Total | 100 |

Production method: The oil-based ingredients were mixed, the clay mineral and the powders were dispersed therein, the separately mixed water-based ingredients were added, and the resulting mixture was emulsified with a homomixer.

## Claims

1. A sunscreen cosmetic comprising:

   (a) 0.5 to 5.0 mass% of 4-tert-butyl-4'-methoxy dibenzoylmethane;
   (b) 3.0 to 10 mass% of ethylhexyl methoxycinnamate;
   (c) 5.0 mass% or more of an amphiphilic substance; and
   (d) a liquid oil containing an ester oil having an IOB of 0.05 to 0.60;

   wherein the blended amounts of the ingredients (a), (b) and (c) fulfill the following expression:

   $$[(a) + (b)]/(c) = 0.5 \text{ to } 3.5$$

2. The cosmetic as in claim 1, wherein the (c) amphiphilic substance is a polyoxyalkylene/polyoxyethylene copolymer dialkyl ether expressed by the following formula (I):

   $$R_1O\text{-}[(AO)_m(EO)_n]\text{-}R_2 \qquad (I)$$

   such that, in formula (I), AO represents an oxyalkylene group having 3 to 4 carbon atoms, EO represents an oxyethylene group, $1 \leq m \leq 70$ and $1 \leq n \leq 70$.

3. The cosmetic as in claim 1 or 2, further comprising one or more other ultraviolet ray absorbing agents chosen from among para-aminobenzoic acid derivatives, salicylic acid derivatives, $\beta,\beta$-diphenyl acrylate derivatives, benzophenone derivatives, benzylidene camphor derivatives, phenyl benzimidazole derivatives, triazine derivatives, phenyl benzotriazole derivatives, anthranil derivatives, imidazoline derivatives, benzalmalonate derivatives, 4,4-diaryl butadiene derivatives and the like.

4. A method for preventing photodegradation of a sunscreen cosmetic, comprising a step of blending, into a sunscreen cosmetic containing (a) 4-tert-butyl-4'-methoxy dibenzoylmethane and (b) ethylhexyl methoxycinnamate, (c) 5.0 mass% or more of an amphiphilic substance and (d) a liquid oil containing an ester oil having an IOB of 0.05 to 0.60.

## INTERNATIONAL SEARCH REPORT

| | International application No. |
| --- | --- |
| | PCT/JP2016/079011 |

| A. CLASSIFICATION OF SUBJECT MATTER |
| --- |
| *A61K8/35*(2006.01)i, *A61K8/37*(2006.01)i, *A61K8/39*(2006.01)i, *A61Q17/04* (2006.01)i |

According to International Patent Classification (IPC) or to both national classification and IPC

| B. FIELDS SEARCHED |
| --- |
| Minimum documentation searched (classification system followed by classification symbols) A61K8/35, A61K8/37, A61K8/39, A61Q17/04 |

| Documentation searched other than minimum documentation to the extent that such documents are included in the fields searched |
| --- |
| Jitsuyo Shinan Koho 1922-1996 Jitsuyo Shinan Toroku Koho 1996-2016 Kokai Jitsuyo Shinan Koho 1971-2016 Toroku Jitsuyo Shinan Koho 1994-2016 |

| Electronic data base consulted during the international search (name of data base and, where practicable, search terms used) |
| --- |

### C. DOCUMENTS CONSIDERED TO BE RELEVANT

| Category* | Citation of document, with indication, where appropriate, of the relevant passages | Relevant to claim No. |
| --- | --- | --- |
| X | JP 2007-106715 A (Shiseido Co., Ltd.), 26 April 2007 (26.04.2007), examples (1-1) to (1-10); comparative examples (1-1) to (1-7); claims; paragraphs [0045], [0064] (Family: none) | 1-4 |
| X | JP 6-256136 A (Nikko Chemicals Co., Ltd.), 13 September 1994 (13.09.1994), example 3; claims; paragraph [0014] (Family: none) | 1,3,4 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 21 October 2016 (21.10.16) | Date of mailing of the international search report 08 November 2016 (08.11.16) |
| --- | --- |
| Name and mailing address of the ISA/ Japan Patent Office 3-4-3,Kasumigaseki,Chiyoda-ku, Tokyo 100-8915,Japan | Authorized officer |
| | Telephone No. |

Form PCT/ISA/210 (second sheet) (January 2015)

**INTERNATIONAL SEARCH REPORT**

International application No.

PCT/JP2016/079011

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2011-157282 A  (Shiseido Co., Ltd.),<br>18 August 2011 (18.08.2011),<br>examples 1 to 4; claims; paragraph [0029]<br>& US 2013/0011348 A1<br>examples 1 to 4; claims; paragraph [0048]<br>& WO 2011/092992 A1     & EP 2529726 A1<br>& CN 102740825 A        & KR 10-2012-0104437 A | 1-4 |
| X | JP 2007-261978 A  (Shiseido Co., Ltd.),<br>11 October 2007 (11.10.2007),<br>blending example 2; claims; paragraph [0021]<br>(Family: none) | 1,3,4 |
| X | JP 9-235216 A  (Shiseido Co., Ltd.),<br>09 September 1997 (09.09.1997),<br>example 11; claims<br>(Family: none) | 1,3,4 |
| A | JP 2004-083541 A  (Shiseido Co., Ltd.),<br>18 March 2004 (18.03.2004),<br>& US 2003/0180335 A1     & EP 1283031 A2<br>& KR 10-2003-0027644 A   & CN 1408339 A | 1-4 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007106701 A

- JP 5540243 B

**Non-patent literature cited in the description**

- Shin-keshohingaku. Nanzando, 2001, 497-504